# FASCICULE DE BREVET EUROPEEN

(11) **EP 3 797 835 B1**
(45) Date de publication et mention de la délivrance du brevet: **07.05.2025**
(21) Numéro de dépôt: 20193782.8
(22) Date de dépôt: 01.09.2020
(51) Int. Cl.: A61K 8/9789, A61K 36/185, A61Q 19/00, A61K 8/34, A61P 17/16

(54) **PRINCIPE ACTIF COSMETIQUE OBTENU PAR BIOCONVERSION PAR LACTOBACILLUS ARIZONENSIS DE SON SUBSTRAT ORIGINEL, PROCEDE D'OBTENTION, COMPOSITION L'INCLUANT ET UTILISATIONS**
KOSMETISCHER WIRKSTOFF, DER AUS DER BIOKONVERSION SEINES URSPRUNGSSUBSTRATS DURCH LACTOBACILLUS ARIZONENSIS ERHALTEN WIRD, HERSTELLUNGSVERFAHREN, DIESEN ENTHALTENDE ZUSAMMENSETZUNG UND SEINE VERWENDUNGEN
COSMETIC ACTIVE INGREDIENT OBTAINED BY BIOCONVERSION BY LACTOBACILLUS ARIZONENSIS OF ITS ORIGINAL SUBSTRATE, METHOD FOR OBTAINING SAME, COMPOSITION INCLUDING SAME AND USES THEREOF

(30) Priorité: 02.09.2019 FR 1909628
(43) Date de publication de la demande: 31.03.2021
(73) Titulaire: Societe Industrielle Limousine d'Application Biologique, 19240 Saint Viance (FR)
(72) Inventeur: PAUFIQUE, Jean, 19130 OBJAT (FR)
(74) Mandataire: Aquinov

(56) Documents cités:
- EP-A1- 3 219 304
- JP-A- 2002 191 387
- US-A1- 2003 186 401

## Description

### Domaine technique

L'invention concerne un principe actif cosmétique comprenant un surnageant de *Lactobacille arizonensis* obtenu par bioconversion par cette bactérie de son substrat originel. L'invention vise également le procédé d'obtention d'un tel principe actif, les compositions cosmétiques l'incluant ainsi que les utilisations cosmétiques de ce principe actif et de ces compositions.

### Etat de l'art

La sècheresse cutanée est un désordre commun affectant de manière égale les hommes et les femmes. Elle s'accompagne de différents symptômes parmi lesquels un inconfort (tiraillements, démangeaisons), une desquamation excessive et l'apparition d'irrégularités inesthétiques (plaques, craquellement, etc.). Bras et mains sont des zones particulièrement sujettes à ce type de désagréments.

L'épiderme, couche la plus superficielle de la peau, est le garant de l'hydratation. Sa capacité à prévenir de la déshydratation dépend de divers paramètres parmi lesquels :
- sa composition en protéines (filaggrine) et lipides.
- l'établissement de jonctions intercellulaires (claudine, desmogléine) pour l'obtention d'une structure cohesive.

C'est au cours du processus de différenciation épidermique que vont être synthétisés les squelettes protéique et lipidique. Les kératinocytes de la couche basale de l'épiderme vont ainsi initier un mécanisme de maturation se traduisant par une migration vers la surface de la peau. Cette étape s'accompagne d'une restructuration profonde de la structure cellulaire, la formation d'un ciment lipidique imperméable et d'une ossature protéique conférant robustesse et résistance à l'épiderme. Au-delà des propriétés mécaniques qu'elle confère, la filaggrine, élément incontournable de l'ossature protéique, participe activement à l'hydratation de la peau en raison de son statut de précurseur à la formation des facteurs d'hydratation naturels. Du bon déroulement du processus de différenciation résulte l'établissement d'une fonction de barrière assurant le « capital eau » de la peau. Soumise en permanence aux assauts de l'environnement, la fonctionnalité de cette barrière est maintenue grâce à son renouvellement continu.

Depuis de nombreuses années, les recherches sur la peau s'intéressent aux mécanismes participant au bon fonctionnement de l'épiderme. Parmi les voies d'approche très récentes, l'influence de la flore cutanée sur l'intégrité de la barrière a fait l'objet de plusieurs travaux. C'est ainsi qu'en dermatologie, les probiotiques sont décrits pour leur capacité à améliorer certains désordres tels que le psoriasis ou la dermatite atopique mais actuellement il n'existe aucun produit satisfaisant pour réguler le microbiote cutané, améliorer le renouvellement de la barrière épidermique et activer les défenses immunes innées.

Ainsi, l'objectif de l'invention est de répondre à ce besoin en proposant un principe actif cosmétique qui appliqué sur une peau saine :
- respecte le microbiote de la peau, c'est-à-dire qui n'engendre pas d'effet sur la diversité et la répartition des communautés bactériennes,
- augmente l'intégrité de la barrière épidermique, en particulier en améliorant la synthèse de l'ossature protéique, en favorisant la cohésion de l'épiderme et en optimisant la formation du ciment lipidique,
- active le renouvellement de la barrière cutanée, en particulier en augmentant la vitesse de renouvellement de l'épiderme,
- optimise l'immunité, en particulier en améliorant la barrière immune de la peau.

Pour y répondre l'invention a pour objet un principe actif cosmétique obtenu par bioconversion par *Lactobacillus arizonensis* de son substrat originel, *Simmondsia chinensis.*

### Résumé de l'invention

L'invention a donc pour objet un postbiotique cosmétique.

Au cours des dix dernières années, les scientifiques ont réalisé de grands progrès dans la compréhension du rôle joué par la flore microbienne sur notre santé. La plupart des travaux ont été réalisés à l'échelle intestinale. Ces études ont conduit d'une part à une meilleure connaissance de l'évolution du microbiote dans divers contextes qu'ils soient physiologiques ou pathologiques et, d'autre part à l'émergence de nouvelles stratégies thérapeutiques. Plusieurs approches ont ainsi vu le jour et par la même occasion ont fait naître la notion de probiotiques. Les probiotiques sont définis comme des microorganismes vivants dont l'administration en quantité adéquate confère des bénéfices pour la santé. Pendant longtemps, l'effet de ces bactéries sur la santé était jugé comme indissociable de leur viabilité. Toutefois, cette théorie semble aujourd'hui évoluer au profit de la notion de postbiotiques, qui correspond aux métabolites bioactifs produits par les probiotiques.

L'influence des postbiotiques sur la qualité de la peau n'a pas été décrite et de façon surprenante, selon l'invention, un principe actif cosmétique obtenu par bioconversion par au moins un lactobacille d'au moins un des substrats dudit lactobacille, c'est-à-dire un postbiotique constitué par des métabolites bioactifs produits par des lactobacilles cultivés en présence de leurs substrats naturels, présente un effet cosmétique sur les peaux saines, en particulier un effet hydratant de la peau.

L'invention vise spécifiquement un principe actif cosmétique caractérisé en ce qu'il s'agit du produit obtenu par bioconversion par *Lactobacillus arizonensis* de *Simmondsia chinensis, Lactobacillus arizonensis* étant une bactérie qui est isolée de *Simmondsia chinensis, Simmondsia chinensis* constituant son substrat originel.

L'invention a également pour objet un procédé d'obtention d'un tel principe actif ainsi que les compositions comprenant au moins 0,1% en poids d'un tel principe actif.

Le principe actif selon l'invention et les compositions l'incluant sont particulièrement utiles pour des utilisations cosmétiques sur des peaux saines pour améliorer la beauté de la peau, notamment pour améliorer l'éclat de la peau et/ou augmenter le rayonnement de la peau et/ou augmenter l'hydratation de la peau. Ainsi un autre objet de l'invention est l'utilisation cosmétique d'un principe actif selon l'invention ou d'une composition l'incluant ainsi qu'un procédé de traitement cosmétique d'une peau saine. D'autres caractéristiques de l'invention ressortiront de la description détaillée qui va suivre.

### Description détaillée de l'invention

### Définitions

Par « actif cosmétique » ou « principe actif cosmétique » ou « principe actif » au sens de l'invention, on entend au moins une molécule, préférentiellement un ensemble de molécules présentant un effet cosmétique sur la peau, c'est-à-dire présentant un effet sur les cellules cutanées.

Par « bioconversion » au sens de l'invention, on entend la transformation des molécules d'une plante en une ou plusieurs autres molécules grâce à l'action d'organismes vivants, comme par exemple un lactobacille.

Par « substrat originel d'un lactobacille » au sens de l'invention, on entend la plante sur laquelle a été isolé et identifié le lactobacille.

### Principe actif cosmétique selon l'invention

L'invention se rapporte à un principe actif cosmétique caractérisé en ce qu'il s'agit du produit obtenu par bioconversion par *Lactobacillus arizonensis* du substrat originel dudit lactobacille.

Préférentiellement, l'invention a pour objet un principe actif cosmétique caractérisé en ce qu'il s'agit du produit obtenu par bioconversion de *Simmondsia chinensis* (le jojoba) par *Lactobacillus arizonensis.* En effet, en utilisant un extrait de *Simmondsia chinensis* comme substrat de *Lactobacillus arizonensis,* il est possible d'avoir un biomimétisme qui se rapproche de l'environnement naturel de *Lactobacillus arizonensis.*

Très préférentiellement le principe actif est obtenu à partir du surnageant obtenu par bioconversion par *Lactobacillus arizonensis* de *Simmondsia chinensis,* c'est-à-dire que le principe actif est constitué en tout ou partie par le surnageant, préférentiellement filtré, obtenu par bioconversion par *Lactobacillus arizonensis* de *Simmondsia chinensis.*

Du métabolisme de la bactérie dépend sa capacité à produire des postbiotiques. Il s'avère donc primordial de calibrer un paramètre fondamental : la nature des apports nutritifs. L'invention consiste donc à enrichir le milieu de culture du Lactobacille (en particulier de *Lactobacillus arizonenzis*) avec un extrait de l'un de ses substrats originels (en particulier de jojoba ou *Simmondsia chinensis* pour *Lactobacillus arizonenzis*), afin de reproduire son environnement naturel et de l'inciter à produire les métabolites nécessaires à sa survie et son adaptation. Avantageusement, les métabolites produits par cette souche ont un effet cosmétique sur la peau saine et notamment peuvent aider au traitement de la sécheresse cutanée des peaux saines.

Le principe actif selon l'invention comprend préférentiellement des sucres. La teneur en sucres totaux (carbohydrates) dans le principe actif peut être déterminée par la méthode de DUBOIS (Dubois M. et al., Analytical chemistry, 28, 3, 350-356, 1956). La teneur en sucres totaux dans le principe actif est exprimée en pourcentage par rapport à la matière sèche. Le principe actif contient entre 1 à 5% de sucres en poids par rapport à la matière sèche.

La composition en sucres simples de la fraction glucidique du principe actif est déterminée par chromatographie liquide ionique et la taille des masses molaires par HPLC avec détection RI. Elle est préférentiellement constituée de glucose, fructose et galactose.

Ces saccharides sont préférentiellement constitués de molécules de masse molaire allant de 360 à 13860 Da.

Préférentiellement, le principe actif selon l'invention comprend au moins un polyol cyclique, et encore plus préférentiellement il comprend au moins du Pinnitol. Les polyols cycliques et en particulier le Pinnitol, participent à l'activité cosmétique du principe actif selon l'invention. La teneur en polyols cycliques et en particulier en Pinnitol est préférentiellement mesurée par chromatographie en phase liquide à haute pression couplée à un spectrophotomètre de masse, en utilisant une courbe de calibration avec un standard : D-Pinnitol commercial, réalisée entre 1 et 1000 ppm.

En plus de polyols cycliques et/ou des sucres, le principe actif selon l'invention peut comprendre d'autres molécules. Il peut notamment comprendre des cendres minérales et/ou des protéines.

La teneur en cendres minérales peut être déterminée par la pesée des résidus issus de l'incinération des échantillons du principe actif selon l'invention à 550°C dans un four à moufle électrique. Préférentiellement, le principe actif selon l'invention présente une teneur en cendres comprise entre 24 à 33% en poids de matières sèches du principe actif. La teneur en protéines peut être déterminée par la méthode de LOWRY (Lowry et al., Protein measurement with the folin reagent, J. Biol. Chem., 193, 265, 1951) ou par le dosage de l'azote total selon la méthode de KJELDHAL (référence : Official method of analysis of the A.O.C., 12th ed. W Horwitz, E.D., New-York, 15-60, 1975). Préférentiellement, le principe actif selon l'invention présente une teneur en protéines comprise entre 5 et 13% en poids de matière sèche, préférentiellement déterminée par la méthode de Kjeldhal.

La teneur en acide lactique peut être déterminée par chromatographie liquide ionique à l'aide d'une gamme d'étalonnage préparée à partir d'un standard commercial. L'acide lactique est sous forme de lactate de sodium lorsque le pH de la solution le contenant est supérieur à son pKa de 3,8. Ainsi, préférentiellement, le principe actif selon l'invention a un pH de 5,4 et présente une teneur en lactate de sodium comprise entre 52 et 85%.

Le principe actif selon l'invention peut se présenter sous forme liquide. Dans ce cas il peut être complémenté par au moins un stabilisant et/ou un conservateur.

Préférentiellement, lorsqu'il est sous forme liquide, le taux de matières sèches du principe actif selon l'invention est compris entre 50 et 150 g/l, préférentiellement entre 70 et 105 g/l. La forme liquide contient préférentiellement 1 à 5% de sucres, 52 à 85% de lactate de sodium, 24 à 33% de cendres minérales et 5 à 13% de protéines, les pourcentages étant donnés en poids de matières sèches. Sous forme liquide il peut être d'une couleur beige à jaune. Toutefois, il peut être décoloré selon des techniques usuelles du domaine.

Le principe actif selon l'invention peut se présenter sous forme solide, notamment sous forme de poudre. Dans ce cas il est préférentiellement constitué par le produit de la bioconversion du substrat par le lactobacille selon l'invention et par un support tel que par exemple la maltodextrine. Préférentiellement, le produit de la bioconversion représente 10 à 50% en poids du principe actif et le support de 50 à 90%. La forme poudre contient donc préférentiellement 50 à 91% de sucres, 5 à 43% de lactate de sodium, 2 à 17% de cendres et 0,5 à 6,5% de protéines, les pourcentages étant donnés en poids du principe actif incluant un support maltodextrine.

### Procédé d'obtention du principe actif selon l'invention

Le principe actif selon l'invention peut être obtenu par tout type de procédé de bioconversion d'au moins un substrat par *Lactobacillus arizonensis.*

Selon un mode de réalisation préféré, l'invention a pour objet un procédé d'obtention d'un principe actif selon l'invention, comprenant la mise en œuvre des étapes suivantes :
- Culture de *Lactobacillus arizonensis,* dans un milieu de culture supplémenté en *Simmondsia chinensis,*
- Arrêt de la culture par inactivation thermique
- Séparation de la biomasse du surnageant par toutes techniques connues de l'homme du métier, par exemple par centrifugation, filtration ou décantation
- préférentiellement filtration du surnageant pour obtenir un liquide comprenant les métabolites postbiotiques constituant le principe actif selon l'invention.

Le procédé selon l'invention peut comprendre également une étape de purification et/ou une étape de décoloration et/ou une étape de désodorisation.

Certaines étapes des procédés décrites ci-avant, prises individuellement, sont usuelles dans le domaine des extractions d'actifs à partir de matières premières naturelles et l'homme du métier est à même d'en ajuster les paramètres réactionnels sur la base de ses connaissances générales.

Le principe actif peut ensuite éventuellement être associé à un support et séché avec ou sans support (atomisation ou lyophilisation notamment), pour se présenter sous forme solide.

Par milieu de culture, on entend tout milieu comprenant au moins les nutriments nécessaires pour la croissance et la multiplication des lactobacilles. L'homme du métier sait qu'un milieu de culture adéquat comprend des nutriments glucidiques, de préférence sous forme d'oligosaccharides, des nutriments azotés, de préférence apportés sous forme de peptides par un extrait de levure, ainsi qu'un apport de sels nécessaire au pouvoir tampon du milieu. Par exemple, le milieu de culture peut être celui de la publication (De MAN, J. C., ROGOSA, M. and SHARPE, M. E. (1960)).

### Utilisation cosmétique

Le principe actif selon l'invention est particulièrement efficace pour un traitement cosmétique sur peau saine donc un traitement non thérapeutique.

En particulier, le principe actif selon l'invention présente une action efficace seul ou dans une composition cosmétique, sur les peaux saines pour améliorer la beauté de la peau. L'invention a donc pour objet l'utilisation cosmétique d'un principe actif selon l'invention ou d'une composition cosmétique en contenant sur une peau saine, pour améliorer la beauté de la peau.

Le principe actif selon l'invention est capable de réguler le microbiote cutané, d'améliorer l'intégrité et le renouvellement de la barrière épidermique, d'activer les défenses immunes innées et de diminuer l'inflammation et la prolifération épidermale anormale :
- respect du microbiote : l'application d'un principe actif selon l'invention sur la peau n'engendre aucun effet sur la diversité et la répartition des communautés bactériennes traduisant la neutralité du principe actif vis-à-vis de la flore cutanée,
- Intégrité de la barrière épidermique augmentée : le principe actif selon l'invention active les mécanismes inhérents à l'établissement d'une fonction barrière efficace. Il améliore la synthèse de l'ossature protéique (filaggrine notamment), favorise la cohésion de l'épiderme (claudine-4 et desmogléine-1 notamment) et optimise la formation du ciment lipidique (expression des enzymes de synthèse des lipides GBA, ABCA12, FASN notamment). Cette action se traduit par une amélioration de la fonction barrière de 71%. Cet effet a également été visualisé sur volontaires.
- Renouvellement de la barrière activé : la vitesse de renouvellement de l'épiderme est accélérée grâce à l'application du principe actif selon l'invention.
- Immunité optimisée : le principe actif selon l'invention permet une amélioration de la barrière immune notamment en augmentant l'expression du récepteur du danger TLR2 et du peptide antimicrobien HBD2.
- Inflammation et prolifération épidermale anormale diminuées : le principe actif selon l'invention permet de diminuer la sécrétion de IL-17 par les lymphocytes T helper 17.

L'invention a donc également pour objet l'utilisation cosmétique spécifique du principe actif tel que décrit dans la présente demande pour :
- réguler l'équilibre de la flore cutanée, et/ou
- renforcer l'intégrité de la barrière cutanée, et/ou
- activer les renouvellements épidermiques, et/ou
- dynamiser les défenses immunes innées.

Ainsi, le principe actif selon l'invention peut être utilisé pour :
- améliorer l'éclat de la peau, et/ou
- augmenter le rayonnement de la peau, et/ou
- augmenter l'hydratation de la peau.

Avantageusement le principe actif selon l'invention permet d'accroître la qualité des peaux souffrant de sécheresse en agissant sur les différents leviers responsables de l'homéostasie épidermique : l'hydratation est améliorée et l'éclat du teint ravivé, et ce sur différentes zones du corps (main et visage en particulier).

Grâce à son action postbiotique, le principe actif selon l'invention améliore l'intégrité et le renouvellement de la barrière épidermique tout en respectant l'équilibre de la flore microbienne.

### Composition cosmétique

Le principe actif selon l'invention est préférentiellement utilisé dans des compositions comprenant un milieu cosmétiquement acceptable. Il s'agit de compositions dans différentes formes galéniques, adaptées à une application par voie topique sur la peau.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect de crèmes, émulsions ou gels ou tous autres aspects des cosmétiques de soin peau.

Il peut s'agir de compositions comprenant au moins 0,1% du principe actif liquide selon l'invention, préférentiellement entre 0,5 et 10% ou au moins 0,02% du principe actif solide selon l'invention, préférentiellement entre 0,1% et 2%.

Ces compositions comprennent, outre le principe actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA *(International Cosmetic Ingredient Dictionary and Handbook* publié par le *Personal Care Product Council).*

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées sur des peaux saines, en particulier des peaux saines sèches et/ou sans éclat pour améliorer l'éclat de la peau et/ou augmenter le rayonnement de la peau et/ou augmenter l'hydratation de la peau.

L'invention vise ainsi également un procédé cosmétique (non thérapeutique), à savoir un procédé cosmétique de traitement d'une peau saine pour améliorer la beauté de la peau, et en particulier pour améliorer l'éclat de la peau et/ou augmenter le rayonnement de la peau et/ou augmenter l'hydratation de la peau, qui consiste en l'application topique sur une peau saine d'une composition comprenant un principe actif selon l'invention.

Préférentiellement la composition comprenant le principe actif cosmétique selon l'invention est appliquée au moins une fois par jour pendant au moins 15 jours.

Afin d'illustrer ces effets cosmétiques sur la qualité de la peau, en particulier sur les propriétés de la peau, les exemples suivants ainsi que des résultats d'essais sont présentés ci-dessous.

### Exemples

### Exemple 1 : Principe actif selon l'invention

L'obtention du principe actif de l'exemple 1 est réalisée à partir de la bactérie *Lactobacillus arizonensis* et d'un extrait de jojoba *Simmondsia chinensis.*

Réalisation de l'extrait de jojoba : Un extrait de tourteau de jojoba est réalisé par hydrolyse enzymatique de tourteau de jojoba solubilisé préalablement dans l'eau. Ce substrat d'origine du *L. arizonensis* est ajouté sous une forme que la bactérie peut assimiler.

Conduite de la culture : Pour la croissance de la bactérie, le milieu de culture est similaire à celui décrit comme MRS végétal vendu par Biokar diagnostics. A ce milieu de culture est ajouté l'extrait de jojoba.

La souche de *L. arizonensis* est donc mise en culture dans le milieu de culture décrit ci-dessus. L'ensemencement est adapté en volume et en temps de croissance à la taille du bioréacteur utilisé.

La culture est réalisée dans les conditions adéquates pour la souche. Le pH est maintenu au cours de la croissance par ajout de soude.

La croissance est suivie par mesure de l'absorbance, par l'ajout de soude et par la consommation de glucose.

A la récolte, la culture est inactivée thermiquement 30 minutes à 80°C, puis centrifugée pour séparer biomasse et surnageant.

Le surnageant est utilisé comme principe actif après filtration et filtration stérilisante.

Le principe actif obtenu par bioconversion, présente les caractéristiques analytiques suivantes :
- La teneur en matière sèche de 80g/L
- Une teneur en carbohydrates de 3,4 g/L (déterminée par la méthode de DUBOIS), soit 4% par rapport à la matière sèche. La fraction carbohydrates est composée majoritairement (67%) de sucres liés contenant du glucose et galactose et 33% de fructose sous forme libre.
- 9,9 g/L de protéines (dosé par la méthode de Kjeldhal), soit 12% par rapport à la matière sèche. La fraction protéique est composée de 65% d'oligopeptides de 243Da à 2000Da.
- 21,5 g/L de cendres (déterminée par la pesée des résidus issus de l'incinération de l'échantillon à 550°C dans un four à moufle électrique), soit 27% de la matière sèche.
- Une teneur en acide lactique de 44,6 g/L, soit 56% de la matière sèche.
- Une teneur en pinnitol de 500 ppm.
- Un pH de 5,4.

### Exemple 2 : Principe actif selon l'invention sous forme solide

Le principe actif selon l'exemple 2 est obtenu à partir de la bactérie *Lactobacillus arizonensis* de la même façon que pour l'exemple 1. Après récupération du surnageant, celui-ci est décoloré, désodorisé, concentré, débactérisé et atomisé sur un support de 75% de maltodextrine.

Le principe actif obtenu, présente les caractéristiques analytiques suivantes :
- Une teneur en matière sèche de 970mg/g
- Une teneur en carbohydrates de 723 mg/g (déterminée par la méthode de DUBOIS) soit 75% par rapport à la matière sèche.
- 16 mg/g de protéines (dosé par la méthode de Kjeldhal) soit 2% par rapport à la matière sèche.
- 89 mg/g de cendres (déterminée par la pesée des résidus issus de l'incinération de l'échantillon à 550°C dans un four à moufle électrique) soit 9% par rapport à la matière sèche.
- Une teneur en acide lactique de 131 mg/g soit 14% par rapport à la matière sèche.
- Une teneur en pinnitol de 1584 ppm.
- Un pH de 5,5.

### Exemple 3 : Produit hors invention obtenu avec un substrat non spécifique du lactobacille L. arizonensis (Extrait de tourteau de tournesol)

Le produit selon l'exemple 3 est obtenu à partir de la bactérie *Lactobacillus arizonensis* de la même façon que pour l'exemple 1 à l'exception de l'extrait de Jojoba qui est remplacé par un extrait de tourteau de tournesol dans des quantités identiques.

Le produit obtenu, présente les caractéristiques analytiques suivantes :
- Une teneur en matière sèche de 79,4 g/L.
- Une teneur en carbohydrates de 2,1 g/L (déterminée par la méthode de DUBOIS), soit 3% par rapport à la matière sèche.
- 11,6 g/L de protéines en poids de matière sèche (dosée par la méthode de Kjeldhal), soit 15%.
- 20,4 g/L de cendres (déterminée par la pesée des résidus issus de l'incinération de l'échantillon à 550°C dans un four à moufle électrique), soit 26%.
- Une teneur en acide lactique de 44,5 g/L, soit 56% par rapport à la matière sèche.
- Un pH de 5,6.

### Exemple 4 : Produit hors invention correspondant au milieu de culture contenant l'extrait de jojoba.

Le produit selon l'exemple 4 correspond au milieu de culture de l'exemple 1 décrit ci-dessus.

Le produit obtenu, présente les caractéristiques analytiques suivantes
- Une teneur en matière sèche de 36,6 g/L.
- Une teneur en carbohydrates de 8,1 g/L (déterminée par la méthode de DUBOIS), soit 22% par rapport à la matière sèche.
- 19,0 g/L de protéines en poids de matière sèche (dosées par la méthode de LOWRY), soit 52% par rapport à la matière sèche.
- 9,5 g/L de cendres (déterminée par la pesée des résidus issus de l'incinération de l'échantillon à 550°C dans un four à moufle électrique), soit 26% par rapport à la matière sèche.

### Exemple 5 : Produit hors invention obtenu en absence du substrat spécifique au lactobacilles (sans jojoba)

Le produit selon l'exemple 5 est obtenu à partir de la bactérie *Lactobacillus arizonensis* de la même façon que pour l'exemple 1 à l'exception du milieu de culture qui ne contient aucun extrait de Jojoba.

Le principe actif obtenu, présente les caractéristiques analytiques suivantes :
- Une teneur en matière sèche de 98,9 g/L
- Une teneur en carbohydrates de 2,6 g/L (déterminée par la méthode de DUBOIS), soit 3% par rapport à la matière sèche. La fraction carbohydrate est constituée à 51% de fructose sous forme libre, et 49% de sucres liés contenant du glucose et du galactose.
- 12,8 g/L de protéines en poids de matière sèche (dosées par la méthode de LOWRY), soit 13% par rapport à la matière sèche.
- 15,8 g/L de cendres (déterminée par la pesée des résidus issus de l'incinération de l'échantillon à 550°C dans un four à moufle électrique), soit 16% par rapport à la matière sèche.
- Une teneur en acide lactique de 67 g/L, soit 68% par rapport à la matière sèche.

### Exemple 6 : exemple de composition soin bonne mine, un soin enlumimeur pour révéler en douceur l'éclat de la peau

La composition de cet exemple est un soin léger et glissant à effet bonne mine immédiat grâce aux nacres roses et dorées. Facile à étaler, il pénètre rapidement en laissant un fini doux et poudré.

Sa formule est présentée dans le Tableau 1.

**[Tableau 1]**

| PHASE | INGREDIENTS - INCI | Fournisseur | % |
|---|---|---|---|
| A | Aqua | - | Qsp 100 |
| | Glycerin | - | 2 |
| | Hydroxyethylcellulose | Ashland / IMCD | 0,5 |
| B | Disodium cetearyl sulfosuccinate | BASF | 0,5 |
| | Glycol palmitate | Seppic | 2 |
| | C10-18 Triglycerides | Stéarinerie Dubois | 1 |
| | Isopropyl myristate | Stéarinerie Dubois | 5 |
| | Dimethicone | Dow / Univar | 2 |
| | Triheptanoin | Seppic | 5 |
| C | Butylene glycol | - | 10 |
| | Titanium dioxide & synthetic fluorphlogopite | SunChemical / Maprecos | 1,5 |
| | Titanium dioxide & synthetic fluorphlogopite | SunChemical / Maprecos | 1,5 |
| D | Ammonium acryloyldimethyltaurate/VP | Clariant | 0,75 |
| E | PRINCIPE ACTIF de l'exemple 2 | Silab | 1 |
| F | Citric acid | - | Qsp pH 5,0 - 5,5 |

La composition est obtenue en mettant en œuvre le mode opératoire suivant :
- Placer A sous agitation magnétique et chauffer à 80°C
- Placer B sous agitation magnétique et chauffer à 80°C.
- Sous rotor-stator, émulsionner B dans A.
- A 40°C, sous agitation modérée, ajouter C puis D et E
- Ajuster le pH avec F

La composition se présente sous forme d'une émulsion souple, de couleur blanc nacré et brillante pH= 5,2 avec une Viscosité (B/5rpm) = 20 000 cPs.

### Exemple 7 : Exemple de composition crème velours pour les mains

La composition de cet exemple est une crème nourrissante et régénérante pour les mains. Rapide à pénétrer, elle laisse un film protecteur confortable et non gras.

La formule de la composition est présentée dans le Tableau 2.

**[Tableau 2]**

| PHASE | INGREDIENTS - INCI | Fournisseur | % |
|---|---|---|---|
| A | Aqua | - | Qsp 100 |
| | Glycerin | - | 2 |
| | Butylene glycol | - | 3 |
| | Aqua & Sodium hydroxide | - | 0,3 |
| B | Cetearyl Alcohol & Dicetyl Phosphate & Ceteth-10 Phosphate | Croda | 5 |
| | PPG-3 Benzyl Ether Myristate | Croda | 5 |
| | C12-15 alkyl benzoate | Evonik/Adara | 3 |
| | Dimethicone | Dow/Univar | 2 |
| | Simmondsia Chinensis Seedl Oil (and) Hydrogenated Vegetable Oil | Sophim | 2 |
| | Cera alba | Baerlocher | 2 |
| C | Hydroxyethyl Acrylate/Sodium Acryloyldimethyl Taurate Copolymer & Polyisobutene & PEG-7 Trimethylolpropane Coconut Ether | Seppic | 2 |
| D | PRINCIPE ACTIF de l'exemple 2 | Silab | 1 |

La composition est obtenue en mettant en œuvre le mode opératoire suivant :
- Placer A sous agitation magnétique et chauffer à 80°C
- Placer B sous agitation magnétique et chauffer à 80°C
- Sous rotor-stator, émulsionner B dans A
- A 40°C, sous agitation modérée, ajouter C puis D.

La composition se présente sous forme d'une émulsion épaisse blanche et brillante, avec un pH= 6,0 - 6,3 et une viscosité (D/5rpm) = 145 000 cPs.

### Exemple 8 : Exemple de composition sous forme de crème pour les pieds

La composition de cet exemple est une crème réconfortante à la texture riche et onctueuse. Rapidement absorbée, elle apporte douceur et souplesse à la peau des pieds sans laisser de film gras.

La formule de la composition est présentée dans le Tableau 3.

**[Tableau 3]**

| PHASE | INGREDIENTS - INCI | Fournisseur | % |
|---|---|---|---|
| A | Aqua | - | Qsp 100 |
| | Butylene glycol | - | 5 |
| B | Cetearyl alcohol & Ceteareth- | Seppic | 5 |
| | Isocetyl stearoyl stearate | Stéarinerie Dubois | 3 |
| | Caprylic capric triglycerides | Stéarinerie Dubois | 7 |
| | Coco-caprylate/caprate | Stéarinerie Dubois | 4 |
| | Dimethicone | Dow / Univar | 2 |
| C | Hydroxyethyl Acrylate/Sodium Acryloyldimethyl Taurate Copolymer & Polyisobutene | Seppic | 2 |
| D | PRINCIPE ACTIF exemple 1 | Silab | 2.5 |

La composition peut être obtenue par la mise en œuvre du mode opératoire suivant :
- Placer A sous agitation magnétique et chauffer à 80°C.
- Placer B sous agitation magnétique et chauffer à 80°C.
- Sous rotor-stator, émulsionner B dans A
- A 40°C, sous agitation modérée, ajouter C puis D

La composition se présente sous forme d'une émulsion épaisse, blanche et brillante, avec un pH= 5,3 - 5,5 et une viscosité (D/5rpm) = 100 000 - 110 000 cPs.

Exemple 9 : Exemple de composition sous forme de lait corporel

La composition de cet exemple est un lait de corps nourrissant, relipidant et protecteur. Facile à étaler et rapide à pénétrer, il laisse la peau douce et soyeuse pour toute la journée.

La formule de la composition selon l'invention est présentée dans le Tableau 4.

**[Tableau 4]**

| PHASE | INGREDIENTS - INCI | Fournisseur | % |
|---|---|---|---|
| A1 | Aqua | - | Qsp 100 |
| | Glycerin | - | 2 |
| | Butylene glycol | - | 5 |
| | Acacia senegal gum & xanthan gum | Seppic | 0,5 |
| A2 | Potassium cetyl phosphate | DSM/IES | 2 |
| B | Isocetyl stearoyl stearate | Stéarinerie Dubois | 2 |
| | Coco-caprylate/caprate | Stéarinerie Dubois | 5 |
| | Dimethicone | Dow/Univar | 2 |
| | Tocopherol & Heliantus Annus Seed Oil | BTSA/CQ Masso | 0.05 |
| | Butyrospermum Parkii (Shea) Butter | Sophim | 2 |
| | Cera alba | Baerlocher | 1 |
| C | Polyacrylate Crosspolymer-6 | Seppic | 1 |
| D | PRINCIPE ACTIF Exemple 1 | Silab | 2.5 |

La composition peut être obtenue par la mise en œuvre du mode opératoire suivant :
- Placer A1 sous agitation modérée. Agiter jusqu'à homogénéisation.
- Ajouter A2 dans A1 sous agitation. Chauffer à 80°C.
- Ajouter A3 dans A1+A2 sous agitation.
- Placer B sous agitation magnétique et chauffer à 80°C.
- Sous rotor-stator, émulsionner B dans A.
- A 40°C, sous agitation modérée, ajouter C puis D.

La composition se présente sous forme d'une émulsion souple blanche et brillante, avec un pH= 6,2 et une viscosité (B/5rpm) = 16 000 cPs.

### Essais démontrant l'efficacité cosmétique de l'invention

### Essais in vitro

### Effet d'un principe actif selon l'invention sur la différenciation / cohésion épidermique

L'objectif de cette étude est d'évaluer la capacité d'un principe actif selon l'invention à maintenir une différenciation / cohésion épidermique fonctionnelle en stimulant les synthèses de filaggrine, de claudine-4 et de desmogléine-1.

La barrière cutanée protège l'organisme contre les contraintes externes qu'elles soient de nature physique, chimique ou environnementale. Elle permet également de lutter contre la déshydratation en limitant la diffusion de l'eau hors de l'organisme. Une multitude de composants protéiques est indispensable à l'établissement de cette fonction barrière comme :
- la filaggrine qui joue un rôle majeur dans la formation du stratum corneum, ultime barrière de notre corps face à l'environnement extérieur. Elle assure également son hydratation grâce aux acides aminés, issus de sa protéolyse, qui constituent une grande partie des « facteurs naturels d'hydratation » ;
- la claudine qui est un des principaux constituants des jonctions serrées : structures complexes qui établissent une véritable barrière intercellulaire dans les couches supérieures de l'épiderme, limitant et régulant le passage des solutés dans cet espace ;
- la desmogléine-1 qui est une protéine d'ancrage appartenant à la famille des cadhérines desmosomales. Elle assure la cohésion entre les kératinocytes adjacents formant ainsi un réseau transcellulaire.

Deux études sont réalisées : une première étude par immunohistofluorescence sur des épidermes reconstruits SlLABSKlN^{®} RE normaux et soumis à un stress hydrique et une seconde étude par immunomarquage sur kératinocytes humains normaux et soumis à un environnement sec.

Etude sur des épidermes reconstruits.

Le protocole opératoire est décrit en suivant.

### I. Culture et traitement des SILABSKIN^{®} RE :

A J0 : Les kératinocytes humains normaux sont ensemencés sur des inserts puis incubés à 37°C dans une atmosphère contenant 5% de CO2.

Le milieu de culture est changé régulièrement.

Après plusieurs jours de culture : un stress hydrique est appliqué ( environnement à 40% d'humidité relative) afin d'induire la déshydratation. Des épidermes reconstruits témoins sont laissés dans un environnement saturé en humidité (82% d'humidité relative).
- Les SlLABSKlN^{®} RE sont traités pendant 24 heures en topique avec le principe actif de l'exemple 1, à 0,25% et 0,50% (V/V).

Après un jour, Les SlLABSKlN^{®} RE sont récupérés, fixés, déshydratés et inclus en paraffine. Des coupes sont ensuite réalisées à l'aide d'un microtome.

### II. Analyse de la synthèse de filaggrine, claudine-4 et desmogléine-1 par immunohistofluorescence :

- Au moyen d'anticorps primaires :
   * Anticorps anti-filaggrine
   * Anticorps anti-claudine-4
   * Anticorps anti-desmogléine-1
- Anticorps secondaires : couplés Alexa Fluor^{®} 488

### III. Lecture des immunomarquages :

La visualisation est réalisée à l'aide d'un microscope couplé à un système d'analyse d'images.

Le taux des différents marqueurs synthétisés est proportionnel à l'intensité de fluorescence verte présente sur les épidermes reconstruits. Une analyse quantitative des images a été réalisée à l'aide d'un logiciel. Les résultats sont exprimés en unités arbitraires (UA).

Les résultats sont donnés dans le Tableau 5 (Capacité d'un principe actif selon l'invention à restaurer la synthèse de filaggrine, claudine-4 et desmogléine-1 dans des SILABSKIN^{®} RE soumis à un stress hydrique).

**[Tableau 5]**

| | Synthèse de filaggrine (UA) | Capacité à restaurer la synthèse de filaggrine (%) | Synthèse de claudine-4 (UA) | Capacité à restaurer la synthèse de claudine-4 (%) | Synthèse de desmogléine-1 (UA) | Capacité à restaurer la synthèse de desmogléine-1 (%) |
|---|---|---|---|---|---|---|
| SlLABSKlN^{®} RE normaux | | | | | | |
| Témoin | 1144 | | 213 | | 648 | |
| Exemple 2 0,50% | 1146 | | 224 | | 747 | |

| SILABSKIN^{®} RE - stress hydrique | | | | | | |
|---|---|---|---|---|---|---|
| Témoin | 862 | | 154 | | 498 | |
| Exemple 2 0,25% | 1067 | 73 | 185 | 53 | 644 | 97 |
| Exemple 2 0,50% | 1138 | 98 | 211 | 97 | 742 | 163 |

Ces résultats montrent que, soumis à un stress hydrique, les SILABSKIN^{®} RE présentent une altération de la différentiation et de la cohésion épidermique, caractérisée par une diminution significative de la synthèse de filaggrine, de claudine-4 et de desmogléine-1. En outre, on constate que, testé à 0,50 % sur ce modèle, le principe actif selon l'invention est capable de restaurer significativement la synthèse de :
- filaggrine : +98% ;
- claudine-4 : +97% ;
- desmogléine-1 : +163%.

Ainsi, l'utilisation d'un principe actif selon l'invention permet de restaurer le processus de différenciation épidermique pour l'obtention d'une structure cohésive.

La seconde étude sur les kératinocytes permet de comparer l'effet des produits des différents exemples 1 à 5. Pour cela leur capacité à restaurer la synthèse de filaggrine par des kératinocytes humains soumis à un environnement sec est comparée. Cette étude a été réalisée par immunocytologie sur des kératinocytes humains normaux soumis à un environnement sec. Le protocole opératoire est le suivant :
Les kératinocytes humains normaux sont ensemencés et incubés à 37°C dans une atmosphère contenant 5% de CO₂ pendant plusieurs jours.

Ensuite, les kératinocytes sont soit incubés à 37°C sous 5% de CO2 dans un environnement à 40% d'humidité relative afin de mimer un environnement sec ou soit conservés dans un environnement saturé en humidité (82% d'humidité relative) en présence ou non des produits des exemples 1 à 5 ou en présence d'une solution de lactate de sodium à 45g/L (pH5,4).

Après 24h, l'immunomarquage est réalisé à l'aide d'un anticorps primaire anti-filaggrine et d'un anticorps secondaire couplé Alexa Flor^{®} 488.

Les noyaux sont colorés avec une solution de DAPI.

La visualisation est réalisée à l'aide d'un microscope IX 70 (Olympus) couplé à un système d'analyse d'images (logiciel NIS-Elements, Nikon).

La filaggrine synthétisée est proportionnelle à l'intensité de fluorescence verte présente sur les kératinocytes. Une analyse quantitative des images a été réalisée à l'aide du logiciel Matlab^{®}. Les résultats sont exprimés en unités arbitraires (UA) dans le Tableau 6 (Effet des différents exemples sur leur capacité à restaurer la synthèse de filaggrine dans des kératinocytes humains soumis à un stress hydrique).

**[Tableau 6]**

| | **Synthèse de filaggrine (UA)** | **Capacité à restaurer la synthèse de filaggrine (%)** |
|---|---|---|
| Kératinocytes normaux | | |
| Témoin | 3 524 | |

| Kératinocytes - stress hydrique | | |
|---|---|---|
| Témoin | 722 | |
| Exemple 1 à 0,5% | 3839 | 111 |
| Exemple 2 à 0,2% | 3526 | 100 |
| Exemple 3 à 0,5% | 609 | 0 |
| Exemple 4 à 0,5% | 653 | 0 |
| Exemple 5 à 0,5% | 700 | 0 |
| Lactate de sodium (45g/L) à 0,5% | 909 | 7 |

Ces résultats montrent que seuls les principes actifs selon l'invention des exemples 1 et 2 correspondants à l'invention permettent une restauration de la synthèse de filaggrine par les cultures de kératinocytes humains soumis à un stress hydrique.

Le produit de l'exemple 3, c'est-à-dire le produit issu de la bioconversion de l'extrait de tournesol par le lactobacille, ne permet pas la restauration de la synthèse de filaggrine. Donc les molécules agissant sur cette synthèse ne sont pas présentes dans le surnageant de culture de lactobacille cultivé dans un milieu de culture supplémenté par un autre substrat que celui dudit lactobacille.

Le produit de l'exemple 4, c'est-à-dire le milieu de culture spécifique du lactobacille ne restaure pas la synthèse de filaggrine. Donc les molécules agissant sur cette synthèse ne sont pas contenues dans le milieu de culture du lactobacille.

Le produit de l'exemple 5, c'est-à-dire le produit issu de la culture du lactobacille dans le milieu de culture « classique » ne restaure pas la synthèse de filaggrine. Donc les molécules agissant sur cette synthèse ne sont pas présentes dans le surnageant d'une culture de lactobacille dans un milieu de culture « classique ».

La solution de lactate de sodium à la même concentration que celle de l'exemple 1 ne restaure pas la synthèse de filaggrine. Donc le lactate de sodium n'est pas responsable de la synthèse de fillagrine dans les cultures de kératinocytes.

### Effet d'un principe actif selon l'invention sur les acteurs majeurs de la synthèse et de la sécrétion des lipides épidermiques

L'objectif de cette étude est d'évaluer la capacité d'un principe actif selon l'invention à restaurer l'expression de différents acteurs indispensables à la synthèse des lipides épidermiques.

Les lipides intercornéocytaires jouent un rôle fondamental dans la fonction barrière cutanée. Essentiellement synthétisée par les kératinocytes, la matrice lipidique présente une composition et une organisation spécifique qui permet d'assurer l'étanchéité de la couche cornée en régulant le flux des électrolytes de l'intérieur de l'organisme vers l'extérieur et inversement. Leurs synthèses et sécrétions impliquent différents acteurs dont :
- la β glucosylceramidase (GBA) est une protéine lysosomale qui catalyse la production des céramides : lipides majoritaires et indispensables au stratum corneum ;
- la fatty acide synthase (FASN) est une enzyme nécessaire à la synthèse des acides gras libres du ciment intercornéocytaire ;
- l'ATP binding cassette subfamily A member 12 (ABCA12) est une molécule clef dans la génération, le transport et la sécrétion des lipides. Elle est également impliquée dans la différenciation kératinocytaire.

Cette étude a été réalisée par PCR sur des épidermes reconstruits SILABSKIN^{®}RE normaux et soumis à un stress hydrique.

Le protocole opératoire de l'étude est décrit en suivant :
Culture et traitement des SlLABSKlN^{®} RE : dans un premier temps, les épidermes reconstruits sont cultivés et traités.
- Les kératinocytes humains normaux sont ensemencés sur des inserts puis incubés à 37°C dans une atmosphère contenant 5% de CO2.
- Le milieu de culture est changé régulièrement.
- Après plusieurs jours de culture, les SlLABSKlN^{®} RE sont soit incubés à 37°C dans un environnement à 40% d'humidité relative pendant plusieurs heures afin d'induire la déshydratation ou soit laissés dans un environnement saturé en humidité (82% d'humidité relative)
- Ensuite, les SILABSKlN^{®} RE sont traités en topique avec le principe actif de l'exemple 1 à 0,25% et 0,50% (V/V)

Enfin, les SlLABSKlN^{®} RE sont récupérés et les ARN totaux extraits.

### II. Analyse de l'expression de GBA, FASN et ABCA12 par PCR quantitative :

Les ARN ont été reverse-transcripts et les ADN complémentaires obtenus ont été analysés par la technique de PCR quantitative. Les ARNm des protéines RPS18 et GUSB, témoins internes de référence, ont été analysés en parallèle des ARNm de GBA, FASN et ABCA12.

La quantification de l'incorporation de fluorescence (SYBR Green) est mesurée en continu à l'aide d'un thermocycleur. L'analyse des Ct (quantification relative) est réalisée à l'aide d'un logiciel.

Les résultats sont donnés dans le Tableau 7 (Capacité d'un principe actif selon l'invention à restaurer l'expression de ABCA12, GBA et FASN dans des SILABSKIN^{®} RE soumis à un stress hydrique).

**[Tableau 7]**

| | Expression de ABCA12 (%) | Capacité à restaurer l'expression de ABCA12 (%) | Expression de GBA (%) | Capacité à restaurer l'expression de GBA (%) | Expression de FASN (%) | Capacité à restaurer l'expression de FASN (%) |
|---|---|---|---|---|---|---|
| SlLABSKlN^{®} RE normaux | | | | | | |
| Témoin | 100 | | 100 | | 100 | |
| Exemple 1 à 0,50% | 153 | | 144 | | 143 | |

| SILABSKIN^{®} RE - stress hydrique | | | | | | |
|---|---|---|---|---|---|---|
| Témoin | 73 | | 65 | | 77 | |
| Exemple 1 à 0,25% | 130 | 211 | 135 | 200 | 125 | 209 |
| Exemple 1 à 0,50% | 139 | 244 | 169 | 297 | 137 | 261 |

Ces résultats montrent que soumis à un stress hydrique, les SILABSKIN^{®} RE présentent une diminution significative des enzymes de synthèse et des protéines de transport des lipides intercornéocytaires. On constate que testé à 0,50 % sur ce modèle, le principe actif selon l'invention restaure significativement l'expression de :
- ABCA12 : +244% ;
- GBA : +297% ;
- FASN : +261%.

En agissant favorablement sur les acteurs majeurs de la biologie des lipides épidermiques, le principe actif selon l'invention active la formation du ciment lipidique, un élément indispensable à la formation d'une barrière fonctionnelle.

### Effet d'un principe actif selon l'invention sur la fonction barrière

L'objectif de cette étude est d'évaluer la capacité d'un principe actif selon l'invention à limiter la Perte Insensible en Eau (PIE) d'un modèle d'épiderme reconstruit SILABSKIN^{®}RE soumis à un stress hydrique.

La perte insensible en eau correspond à la diffusion passive d'eau à travers la couche cornée. Sa mesure permet d'apprécier l'intégrité de la fonction barrière épidermique. Lorsque celle-ci est altérée, le flux d'eau vers le milieu extérieur s'intensifie augmentant la valeur de la PIE.

Cette étude a été réalisée par mesure de la PIE à l'aide d'un Tewamètre^{®} sur des épidermes reconstruits SILABSKIN^{®}RE normaux et soumis à un stress hydrique.

Le protocole opératoire est décrit en suivant :

### I. Culture et traitement des SlLABSKlN^{®} RE :

- les kératinocytes humains normaux sont ensemencés sur des inserts puis incubés à 37°C dans une atmosphère contenant 5% de CO2.
- le milieu de culture est changé régulièrement.
- Après plusieurs jours de culture, les SlLABSKlN^{®} RE sont soit incubés à 37°C sous 5% de CO2 dans un environnement à 40% d'humidité relative pendant plusieurs heures afin d'induire la déshydratation, soit laissés dans un environnement saturé en humidité (82% d'humidité relative).
- Puis, les SlLABSKlN^{®} RE sont traités pendant 24 heures en topique avec le principe actif de l'exemple 1 à 0,25% et 0,50% (V/V).
- Enfin, les SILABSKlN^{®} RE sont asséchés et incubés une heure à température ambiante.

La mesure de la PIE est réalisée à l'aide d'un Tewamètre^{®} CM 820 (Courage & Khazaka).

Les résultats sont donnés dans le Tableau 8 (Capacité d'un principe actif selon l'invention à limiter la perte insensible en eau des SlLABSKlN^{®} RE soumis à un stress hydrique).

**[Tableau 8]**

| | PIE (g/h/m²) | Capacité à limiter la perte insensible en eau (%) |
|---|---|---|
| SlLABSKlN^{®} RE normaux | | |
| Témoin | 10,74 | |
| Exemple 2 à 0,50% | 11,32 | |

| SILABSKIN^{®} RE - stress hydrique | | |
|---|---|---|
| Témoin | 13,77 | |
| Exemple 2 à 0,25% | 12,62 | 38% |
| Exemple 2 à 0,50% | 11,62 | 71% |

Soumis à un stress hydrique, les SlLABSKlN^{®} RE présentent une augmentation significative de 28% de la perte insensible en eau.

Testé à 0,5 % sur ce modèle, le principe actif selon l'invention limite significativement la déshydratation épidermique de 71%.

En conservant une barrière cutanée fonctionnelle, le principe actif selon l'invention limite les effets de la déshydratation épidermique.

### Effet d'un principe actif selon l'invention sur les défenses immunes innées

L'objectif de cette étude est d'évaluer la capacité d'un principe actif selon l'invention (exemple 1) à réguler les défenses immunes innées de la peau. Pour cela l'expression des défensines 2 (HBD2) et du Toll-like Receptor 2 (TLR2) a été mesurée sur un modèle d'épiderme reconstruit SILABSKIN^{®}RE dans un stress hydrique.

Une des principales fonctions de la peau est de former une barrière efficace contre les microbes pathogènes auxquels elle est confrontée continuellement. Les kératinocytes sont les premières sentinelles de cette barrière immune. Ils possèdent des récepteurs, dont le TLR2, qui leur permettent de détecter et de faire la distinction entre les micro-organismes résidents de la flore commensale et les micro-organismes transitoires pathogènes. Pour les neutraliser et les éliminer, les kératinocytes synthétisent des peptides antimicrobiens tels que les HBD2. Ces derniers sont largement exprimés par les kératinocytes et se caractérisent par une action antimicrobienne à large spectre.

Cette étude a été réalisée par QPCR sur des épidermes reconstruits SILABSKIN^{®}RE normaux et soumis à un stress hydrique.

Le protocole opératoire est décrit en suivant.

### I. Culture et traitement des SILABSKIN^{®} RE :

- Les kératinocytes humains normaux sont ensemencés sur des inserts puis incubés à 37°C.
- Le milieu de culture est changé régulièrement.
- Les SILABSKIN^{®} RE sont soit incubés à 37°C sous 5% de CO2 dans un environnement à 40% d'humidité relative pendant plusieurs heures afin d'induire la déshydratation ou soit laissés dans un environnement saturé en humidité (82% d'humidité relative)

Puis, les SlLABSKlN^{®} RE sont traités pendant 24 heures en topique avec le principe actif de l'exemple 1 à 0,25% et 0,50% (V/V) et soit incubés à 37°C sous 5% de CO2 dans un environnement à 40% d'humidité relative ou soit laissés dans un environnement saturé en humidité (82% d'humidité relative).
- Enfin, les SILABSKlN^{®} RE sont récupérés et les ARN totaux extraits.

### II. Analyse de l'expression de HBD2 et TLR2 par PCR quantitative

Les ARN ont été reverse-transcripts et les ADN complémentaires obtenus ont été analysés par la technique de PCR quantitative. Les ARNm des protéines RPS18 et GUSB, témoins internes de référence, ont été analysés en parallèle des ARNm de HBD2 et TLR2.

La quantification de l'incorporation de fluorescence (SYBR Green) est mesurée en continu à l'aide d'un thermocycleur. L'analyse des Ct (quantification relative) est réalisée à l'aide d'un logiciel.

Les résultats sont donnés dans le Tableau 9 (Capacité d'un principe actif selon l'invention à restaurer l'expression de HBD2 et TLR2 par des SlLABSKlN^{®} RE soumis à un stress hydrique).

**[Tableau 9]**

| | Expression de HBD2 (%) | Capacité à restaurer l'expression de HBD2 (%) | Expression de TLR2 (%) | Capacité à restaurer l'expression de TLR2 (%) |
|---|---|---|---|---|
| SlLABSKlN^{®} RE normaux | | | | |
| Témoin | 100 | | 100 | |
| Exemple 2 à 0,50% | 113 | | 144 | |

| SILABSKIN^{®} RE - stress hydrique | | | | |
|---|---|---|---|---|
| Témoin | 43 | | 67 | |
| Exemple 2 à 0,25% | 62 | 33 | 128 | 185 |
| Exemple 2 à 0,50% | 100 | 100 | 147 | 242 |

Ces résultats montrent que soumis à un stress hydrique, les SlLABSKlN^{®} RE présentent une diminution significative des défenses innées anti bactériennes. On constate que testé à 0,50 % sur ce modèle, le principe actif selon l'invention restaure significativement l'expression de :
- HBD2 : +100%,
- TLR2 : +242%

L'utilisation d'un principe actif selon l'invention limite ainsi les effets délétères d'un stress hydrique sur la barrière immune cutanée.

### Effet du principe actif selon l'invention sur la sécretion de l'IL-17 par les lymphocytes TT helper 17

L'interleukine 17 (IL-17) est une cytokine produite par les lymphocytes T helper 17 (Th17L) qui a des effets sur la biologie de l'épiderme. Elle cause une inflammation et une prolifération épidermale anormale. En particulier, l'IL-17 est impliqué dans le psoriasis. Cette étude a été réalisée afin de démontrer l'activité potentielle du principe actif selon l'invention sur la sécrétion de l'IL-17 par les Th17L.

Le protocole est le suivant :
Les lymphocytes T natifs sont isolés de cellules mononuclées du sang périphérique et cultivés dans un milieu contenant un mélange permettant leur différenciation en Th17L. Les cellules sont ensuite incubées à 37°C dans une atmosphère contenant 5% de CO2.

Après plusieurs jours, le milieu de culture est éliminé et remplacé par un milieu contenant un mélange permettant la différenciation en présence ou en absence de cyclosporine ou du principe actif selon l'exemple 2 à 0,1%.

Après plusieurs heures, le surnageant est recueilli et congelé à -80°C.

Les IL-17 sont dosés par test ELISA.

Les résultats sont résumés dans le tableau 10 suivant :

**[Tableau 10]**

| | Sécrétion d'IL-17 (pg/mL) | Capacité à limiter la sécrétion d'IL-17 (%) |
|---|---|---|
| Témoin | 1 497 | |
| Cyclosporine à 1 µg/mL | 567 | -62 |
| Principe actif exemple 2 à 0,1% | 865 | -42 |

Lorsqu'ils sont placés dans un milieu induisant leur différenciation, les lymphocytes T natifs se différencient en Th17L. Ce procédé conduit à une augmentation de la production d'IL-17.

Testé à 0,1% sur des lymphocytes Th17, le principe actif selon l'invention limite la sécrétion d'IL-17 de 42%.

### Effet d'un principe actif selon l'invention sur l'expression de marqueurs dermique

L'objectif de cette étude est d'évaluer l'effet du principe actif selon l'exemple 1 à augmenter l'expression d'ARNm codant pour les collagènes principaux du derme : collagène I et collagène III, ainsi que les collagènes principaux de la jonction dermo épidermique : collagène IV et collagène VII et pour l'enzyme HAS-2 (Hyaluronan synthase 2) impliquée dans la synthèse d'acide hyalyuronique.

Cette étude a été réalisé par PCR sur des fibroblastes humains normaux.

Le protocole opératoire de l'étude est décrit en suivant :

### I. Culture et traitement des fibroblastes :

- Les fibroblastes humains normaux sont ensemencés dans un milieu de culture puis incubés à 37°C dans une atmosphère contenant 5% de CO2.
- Ensuite, les fibroblastes sont cultivés en présence ou en absence du principe actif de l'exemple 1 à 0, 5% et 1% (V/V).

Le TGF-β à 10 ng/mL est utilisé comme témoin positif. Enfin, les fibroblastes sont récupérés et les ARN totaux extraits.

### II. Analyse de l'expression de Coll I, Coll III, Coll IV, Coll VII et HAS-2 par PCR quantitative :

Les ARN ont été reverse-transcripts et les ADN complémentaires obtenus ont été analysés par la technique de PCR quantitative. Les ARNm de la protéine RPS18, témoin interne de référence, ont été analysés en parallèle des ARNm de Coll I, Coll III, Coll IV, Coll VII et HAS-2.

La quantification de l'incorporation de fluorescence (SYBR Green) est mesurée en continu à l'aide d'un thermocycleur. L'analyse des Ct (quantification relative) est réalisée à l'aide d'un logiciel.

Les résultats sont donnés dans les Tableaux 11, 12 et 13 ci après.

**[Tableau 11]**

| | **Collagène I** | | **Collagène III** | |
|---|---|---|---|---|
| | Expression (%) | Efficacité / témoin (%) | Expression (%) | Efficacité / témoin (%) |
| Témoin | 100 | | 100 | |
| Exemple 1 à 0.5% | 114 | +14% | 115 | +15% |
| Exemple 1 à 1.0% | 122 | +22% | 130 | +30% |
| TGF-β 10 ng/mL | 226 | +126% | 139 | +39% |

**[Tableau 12]**

| | **Collagène IV** | | **Collagène VII** | |
|---|---|---|---|---|
| | Expression (%) | Efficacité / témoin (%) | Expression (%) | Efficacité / témoin (%) |
| Témoin | 100 | | 100 | |
| Exemple 1 à 0.5% | 114 | +14% | 107 | +7% |
| Exemple 1 à 1.0% | 126 | +26% | 121 | +21% |
| TGF-β 10 ng/mL | 318 | +218% | 334 | +234% |

**[Tableau 13]**

| | **HAS-2** | |
|---|---|---|
| | Expression (%) | Efficacité / témoin (%) |
| Témoin | 100 | |
| Exemple 1 à 0.5% | 127 | +27% |
| Exemple 1 à 1.0% | 134 | +34% |
| TGF-β 10 ng/mL | 78 | |

Ces résultats montrent que le principe actif selon l'invention augmente significativement l'expression des marqueurs dermiques. Ainsi, le principe actif selon l'invention augmente l'expression d'ARNm codant pour le collagène I de 22%, le collagène III de 30%, le collagène IV de 26%, le collagène VII de 21% et pour l'enzyme HAS-2 (Hyaluronan synthase 2) de 34%.

### Essais in vivo

Le principe actif de l'exemple 2 a été formulé au sein d'une formule unique pour réaliser l'ensemble des études in vivo, sur panel caucasien et asiatique. La formule est décrite dans le Tableau 14.

**[Tableau 14]**

| | |
|---|---|
| Isononyl isononanoate (Lanol 99, Seppic) | 5,0 |
| Behenyl Alcohol / Arachidyl glucoside / Arachidyl alcohol (Montanov 202, Seppic) | 3,0 |
| Principe actif selon l'invention - exemple 1 | 1 |
| Cetearyl alcohol / cetearyl glucoside (Montanov 68, Seppic) | 2,0 |
| Conservateurs | 1,0 |
| Polyacrylamide / C13-14 isoparaffin / Laureth-7 (Sepigel 305, Seppic) | 0,3 |
| Eau | qsp 100 |

### Effet d'un principe actif selon l'invention sur le microbiote cutané

L'objectif de cette étude est d'évaluer, in vivo, l'effet d'un principe actif selon l'invention formulé à 1% en émulsion sur le microbiote cutané, en comparaison au placebo, après 28 jours d'application.

Cette étude a été réalisée sur 18 volontaires caucasiens sains, âgés de 32 à 52 ans (âge moyen 43 ± 6 ans) et présentant une peau sèche au niveau des mains et du visage. Les volontaires ont appliqué matin et soir la formule contenant le principe actif selon l'invention et le placebo en hémi-visage.

L'influence du principe actif selon l'invention sur le microbiote cutané a été mesurée par séquençage des régions variables V1-V2 du gène codant pour l'ARN ribosomique 16S (ADNr 16S), commun à l'ensemble des bactéries, grâce au système MiSeq^{®} (Illumina). Le prélèvement et analyse du microbiote cutané a été effectué au niveau du niveau du front.

Les paramètres analysés sont :
- l'alpha-diversité qui reflète la diversité au sein de l'échantillon ;
- la taxonomie qui évalue en abondance relative la composition et la répartition des communautés bactériennes. Dans le cas présent, l'analyse a été conduite au niveau du genre.

Un résumé des résultats correspondant à l'effet du principe actif selon l'invention formulé à 1% en émulsion, sur l'alpha-diversité du microbiote cutané, donnée par l'indice de Shannon, sur des volontaires caucasiens est décrit en suivant :

**[Tableau 15]**

| | J0 | J28 |
|---|---|---|
| Indice de Shannon | 1,58 | 1,75 |

Un résumé des résultats correspondant à l'effet du principe actif selon l'invention formulé à 1% en émulsion, sur la répartition des communautés bactériennes au niveau des 10 genres majoritaires, après 28 jours d'application biquotidienne est présenté en suivant :

**[Tableau 16]**

| | Abondance relative (%) | |
|---|---|---|
| | J0 | J28 |
| *Acinetobacter* | 0,9% | 0,9% |
| *Anaerococcus* | 0,4% | 0,7% |
| *Corynebacterium* | 4,9% | 5,4% |
| *Cutibacterium* | 57,1% | 54,1% |
| *Enhydrobacter* | 1,4% | 2,2% |
| *Haemophilus* | 0,2% | 0,4% |
| *Paracoccus* | 0,3% | 0,4% |
| *Prevotella* | 0,3% | 0,4% |
| *Staphylococcus* | 20,3% | 20,7% |
| *Streptococcus* | 1,2% | 1,4% |

Dans les conditions de cette étude, après 28 jours de traitement, le principe actif selon l'invention, formulé à 1%, ne perturbe pas le microbiote des peaux souffrant de sécheresse (pas d'effet significatif, ni sur la diversité, ni sur la composition et la répartition du microbiote cutané).

### Effet d'un principe actif selon l'invention sur la barrière cutanée

L'objectif de cette étude est d'évaluer, in vivo, en comparaison au placebo, l'effet d'un principe actif selon l'invention formulé à 1% en émulsion sur la barrière cutanée.

### Panel caucasien :

Composé de 18 volontaires sains, âgés de 32 à 52 ans (âge moyen 43 ± 6 ans), présentant une peau sèche au niveau des mains et du visage, ayant appliqué matin et soir la formule contenant le principe actif selon l'invention et le placebo en hémi-visage.

### Panel asiatique :

Composé de 31 volontaires sains, âgés de 20 à 64 ans (âge moyen 44 ± 14 ans), présentant une peau sèche au niveau du visage, ayant appliqué matin et soir la formule contenant le principe actif selon l'invention et le placebo en hémi-visage.

La capacité du principe actif selon l'invention à améliorer la qualité de la barrière cutanée a été évaluée selon les méthodes suivantes :
- mesure de la perte insensible en eau (PIE), sur panels caucasien et asiatique, au niveau des joues à l'aide d'un Téwamètre^{®} (Courage & Khazaka) ;
- détermination de la quantité de lipides épidermiques sur panel caucasien à partir d'acquisitions réalisées au niveau des joues par microspectroscopie Raman (Horiba).

Un résumé des résultats correspondant à l'effet du principe actif selon l'invention formulé à 1% en émulsion sur la PIE de volontaires caucasiens et asiatiques est présenté en suivant :
On constate qu'après 28 jours d'application biquotidienne et en comparaison au placebo, le principe actif selon l'invention formulé à 1%, en émulsion, améliore la fonction barrière en diminuant les pertes insensibles en eau au niveau du visage :
- de 15,1% pour le panel caucasien (p = 0,0005), effet observé chez 83% des volontaires ;
- de 20,9% chez les volontaires asiatiques (p < 0,001). Cet effet a été observé chez 87% d'entre eux.

Un résumé des résultats correspondant à l'effet d'un principe actif selon l'invention formulé à 1% en émulsion sur la quantité de lipides épidermiques de volontaires caucasiens est présenté en suivant :
Après 28 jours d'application biquotidienne au niveau du visage et en comparaison au placebo, le principe actif selon l'invention formulé à 1% en émulsion, améliore la fonction barrière en augmentant de façon significative la quantité de lipides dans le stratum corneum de 6,1% (p = 0,0022). Cet effet a été observé chez 67% des volontaires.

### Effet d'un principe actif selon l'invention sur le renouvellement cellulaire

L'objectif de cette étude est d'évaluer, in vivo, en comparaison au placebo, la capacité d'un principe actif selon l'invention formulé à 1% en émulsion à favoriser le renouvellement épidermique.

Cette étude a été réalisée sur 14 volontaires caucasiens sains, âgés de 28 à 52 ans (âge moyen 43 ± 8 ans) et présentant une peau sèche au niveau des mains et du visage. Les volontaires ont appliqué en hémi-bras, matin et soir la formule contenant le principe actif selon l'invention et le placebo sur la face interne des avant-bras.

La capacité du principe actif selon l'invention à améliorer le renouvellement cellulaire a été évaluée à partir de photographies réalisées sur la face interne des avant-bras, à l'aide d'un dermatoscope.

Un résumé des résultats correspondant à l'effet du principe actif selon l'invention formulé à 1% en émulsion sur le renouvellement cellulaire de volontaires caucasiens est présenté en suivant :
Après 14 jours d'application biquotidienne et en comparaison au placebo, le principe actif selon l'invention formulé à 1% en émulsion accélère le renouvellement cellulaire de la peau en diminuant plus rapidement le paramètre b*, représentatif de la couleur jaune mélanique. Ainsi, avec le principe actif selon l'invention, la vitesse de renouvellement est augmentée de plus de 20% (gain de 3 jours sur 14 jours).

### Bénéfices cosmétiques d'un principe actif selon l'invention

L'objectif de cette étude est d'évaluer, in vivo, l'effet d'un principe actif selon l'invention formulé à 1% en émulsion sur la qualité de la peau, en comparaison au placebo, après 28 jours d'application.

Panels caucasiens (présentant une peau sèche au niveau des mains et du visage) :
- Etude au niveau du visage : panel composé de 18 volontaires sains, âgés de 32 à 52 ans (âge moyen 43 ± 6 ans), ayant appliqué matin et soir la formule contenant le principe actif selon l'invention et le placebo en hémi-visage.
- Etude au niveau des mains : panel composé de 19 volontaires sains, âgés de 26 à 52 ans (âge moyen 43 ± 7 ans), ayant appliqué 4 fois par jour la formule contenant le principe actif selon l'invention et le placebo sur le dessus des mains.
- Panel asiatique (présentant une peau sèche au niveau des mains) : Composé de 31 volontaires sains, âgés de 20 à 64 ans (âge moyen 44 ± 14 ans), ayant appliqué matin et soir la formule contenant le principe actif selon l'invention et le placebo en hémi-visage.

La capacité du principe actif selon l'invention à améliorer la qualité de la peau a été évaluée selon les méthodes suivantes :
- mesure du taux d'hydratation à l'aide d'un Cornéomètre^{®} (Courage & Khazaka) au niveau des mains sur panel caucasien et au niveau des joues sur panel asiatique ;
- visualisation de la sécheresse cutanée à l'aide d'un dermatoscope au niveau des mains sur panel caucasien ;
- évaluation de l'éclat du teint par scorage clinique, sur panel caucasien et asiatique ;
- visualisation de l'éclat du teint sur des photographies numériques sur panel caucasien et asiatique ;
- prélèvement et analyse du microbiote cutané au niveau du front sur panel caucasien.

Un résumé des résultats correspondant à l'effet hydratant d'un principe actif selon l'invention formulé à 1% en émulsion est présenté en suivant :
Après 28 jours d'application biquotidienne et en comparaison au placebo, le principe actif selon l'invention formulé à 1%, en émulsion augmente significativement l'hydratation de la peau au niveau :
- des mains des volontaires caucasiens de 16,2% (p = 0,0039). Cet effet a été observé chez 67% des volontaires.
- du visage des volontaires asiatiques de 15,8% (p < 0,001), effet observé chez 87% d'entre eux.

Un résumé des résultats correspondant à l'effet du principe actif selon l'invention formulé à 1% en émulsion sur l'éclat du teint, évalué par experts, sur volontaires caucasiens et sur volontaires asiatiques est présenté en suivant :
Après 28 jours d'application biquotidienne et en comparaison au placebo, le principe actif selon l'invention formulé à 1% en émulsion, améliore l'éclat du teint des volontaires caucasiens et asiatiques.

Chez les volontaires caucasiens, le principe actif selon l'invention :
- augmente significativement le rayonnement de la peau (+7,4%, p 0,0023), la couleur rose représentative d'un teint frais (+14,7%, p = 0,0077), ainsi que l'effet bonne mine (+6,4%, p = 0,0147).
- diminue significativement la couleur olive (-9,2%, p=0,0232)

Chez les volontaires asiatiques, le principe actif selon l'invention augmente significativement le rayonnement de la peau (+13,9% p=0,000), l'homogénéité du teint (+6,0%, p = 0,025), ainsi que l'hydratation cutanée (+7,0%, p = 0,014).

L'utilisation d'un principe actif selon l'invention sur la peau saine améliore donc la beauté de la peau, en particulier son éclat et son hydratation.

## Revendications

1. Principe actif cosmétique **caractérisé en ce qu'**il s'agit du produit obtenu par bioconversion par *Lactobacillus arizonensis* de *Simmondsia chinensis.*

2. Principe actif cosmétique selon la revendication 1, **caractérisé en ce qu'**il est obtenu à partir du surnageant obtenu par bioconversion par *Lactobacillus arizonensis* de *Simmondsia chinensis.*

3. Principe actif cosmétique selon la revendication 1 ou 2, **caractérisé en ce qu'**il s'agit du surnageant obtenu par bioconversion par *Lactobacillus arizonensis* de *Simmondsia chinensis.*

4. Principe actif cosmétique selon l'une des revendications 1 à 3, **caractérisé en ce qu'**il comprend des sucres.

5. Principe actif cosmétique selon la précédente revendication, **caractérisé en ce que** la fraction glucidique du principe actif comprend des polysaccharides et des oligosaccharides de glucose, galactose et fructose.

6. Principe actif selon l'une des précédentes revendications, **caractérisé en ce qu'**il comprend au moins un polyol cyclique.

7. Principe actif cosmétique selon l'une des précédentes revendications, **caractérisé en ce qu'**il comprend au moins du Pinnitol.

8. Principe actif cosmétique selon l'une des précédentes revendications, **caractérisé en ce qu'**il comprend des protéines et/ou des minéraux et/ou de l'acide lactique.

9. Principe actif selon l'une des précédentes revendications, **caractérisé en ce qu'**il se présente sous forme de poudre.

10. Composition cosmétique comprenant au moins 0,1% en poids d'un principe actif selon l'une des revendications 1 à 8.

11. Composition cosmétique selon la précédente revendication, **caractérisée en ce qu'**elle se présente sous forme de de gel, émulsion ou crème.

12. Procédé d'obtention d'un principe actif selon l'une des revendications 1 à 8, **caractérisé en ce qu'**il comprend les étapes suivantes :
- Culture de *Lactobacillus arizonensis* dans un milieu de culture supplémenté de *Simmondsia chinensis*
- Arrêt de la culture par inactivation thermique
- Séparation de la biomasse du surnageant
- Filtration du surnageant.

13. Procédé selon la précédente revendication, **caractérisé en ce qu'**il comprend également une étape de purification et/ou une étape de décoloration et/ou une étape de désodorisation et/ou une étape d'atomisation.

14. Utilisation cosmétique d'un principe actif cosmétique selon l'une des revendications 1 à 8 sur une peau saine pour améliorer la beauté de la peau.

15. Utilisation cosmétique d'une composition cosmétique selon l'une des revendications 10 ou 11, sur une peau saine, pour améliorer la beauté de la peau.

16. Utilisation cosmétique non thérapeutique d'une composition cosmétique selon l'une des revendications 10 ou 11, pour améliorer l'éclat de la peau et/ou augmenter le rayonnement de la peau et/ou augmenter l'hydratation de la peau.

17. Composition cosmétique selon l'une des revendications 10 ou 11 pour son utilisation pour réguler l'équilibre de la flore cutanée et/ou pour renforcer l'intégrité de la barrière cutanée et/ou pour activer les renouvellements épidermiques et/ou pour dynamiser les défenses immunes innées.

18. Procédé de traitement cosmétique d'une peau saine, pour améliorer la beauté de la peau, **caractérisé en ce qu'**il consiste à appliquer au moins une fois par jour sur la peau une composition selon l'une des revendications 10 ou 11.

## Patentansprüche

1. Kosmetischer Wirkstoff, **dadurch gekennzeichnet, dass** es sich um ein Produkt handelt, das durch Biokonversion durch *Lactobacillus arizonensis von Simmondsia chinensis* erhalten wird.

2. Kosmetischer Wirkstoff nach Anspruch 1, **dadurch gekennzeichnet, dass** er aus dem Überstand erhalten wird, der durch Biokonversion durch *Lactobacillus arizonensis von Simmondsia chinensis* erhalten wird.

3. Kosmetischer Wirkstoff nach Anspruch 1 oder 2,
**dadurch gekennzeichnet, dass** es sich um den Überstand handelt, der durch Biokonversion durch *Lactobacillus arizonensis von Simmondsia chinensis* erhalten wird.

4. Kosmetischer Wirkstoff nach einem der Ansprüche 1 bis 3,
**dadurch gekennzeichnet, dass** er mindestens Zucker umfasst.

5. Kosmetischer Wirkstoff nach dem vorstehenden Anspruch,
**dadurch gekennzeichnet, dass** der Kohlenhydratanteil des Wirkstoffs Polysaccharide und Oligosaccharide von Glucose, Galactose und Fructose umfasst.

6. Wirkstoff nach einem der vorstehenden Ansprüche,
**dadurch gekennzeichnet, dass** er mindestens ein zyklisches Polyol umfasst.

7. Kosmetischer Wirkstoff nach einem der vorstehenden Ansprüche, **dadurch gekennzeichnet, dass** er mindestens Pinnitol umfasst.

8. Kosmetischer Wirkstoff nach einem der vorstehenden Ansprüche, **dadurch gekennzeichnet, dass** er mindestens Proteine und/oder Mineralstoffe und/oder Milchsäure umfasst.

9. Wirkstoff nach einem der vorstehenden Ansprüche,
**dadurch gekennzeichnet, dass** er mindestens in Pulverform vorliegt.

10. Kosmetische Zusammensetzung, umfassend mindestens zu 0,1 Gew.-% einen Wirkstoff nach einem der Ansprüche 1 bis 8.

11. Kosmetische Zusammensetzung nach dem vorstehenden Anspruch, **dadurch gekennzeichnet, dass** sie in Form eines Gels, einer Emulsion oder einer Creme vorliegt.

12. Verfahren zum Erhalten eines Wirkstoffs nach einem der Ansprüche 1 bis 8, **dadurch gekennzeichnet, dass** es die folgenden Schritte umfasst:
- Kultivieren von *Lactobacillus arizonensis* in einem Kulturmedium, das mit *Simmondsia chinensis* angereichert ist
- Stoppen des Kultivierens durch thermische Inaktivierung
- Trennen der Biomasse von dem Überstand
- Filtern des Überstands.

13. Verfahren nach dem vorstehenden Anspruch,
**dadurch gekennzeichnet, dass** es auch einen Reinigungsschritt und/oder einen Entfärbungsschritt und/oder einen Desodorierungsschritt und/oder einen Zerstäubungsschritt umfasst.

14. Kosmetische Verwendung eines kosmetischen Wirkstoffs nach einem der Ansprüche 1 bis 8 auf einer gesunden Haut zum Verbessern der Schönheit der Haut.

15. Kosmetische Verwendung einer kosmetischen Zusammensetzung nach einem der Ansprüche 10 oder 11 auf einer gesunden Haut zum Verbessern der Schönheit der Haut.

16. Nichttherapeutische kosmetische Verwendung einer kosmetischen Zusammensetzung nach einem der Ansprüche 10 oder 11 zum Verbessern des Glanzes der Haut und/oder Erhöhen der Ausstrahlung der Haut und/oder Erhöhen der Feuchtigkeit der Haut.

17. Kosmetische Zusammensetzung nach einem der Ansprüche 10 oder 11 zur Verwendung zum Regulieren des Gleichgewichts der Hautflora und/oder zum Stärken der Integrität der Hautbarriere und/oder zum Aktivieren der epidermalen Erneuerungen und/oder zum Mobilisieren der angeborenen Abwehrkräfte.

18. Verfahren zum kosmetischen Behandeln einer gesunden Haut, zum Verbessern der Schönheit der Haut, **dadurch gekennzeichnet, dass** es darin besteht, mindestens einmal täglich eine Zusammensetzung nach einem der Ansprüche 10 oder 11 auf die Haut aufzutragen.

## Claims

1. A cosmetic active substance **characterized in that** it is the product obtained through bioconversion by *Lactobacillus arizonensis* of *Simmondsia chinensis.*

2. The cosmetic active substance according to claim 1,
**characterized in that** it is obtained from the supernatant obtained through bioconversion with *Lactobacillus arizonensis* of *Simmondsia chinensis.*

3. The cosmetic active substance according to claim 1 or 2,
**characterized in that** it is the supernatant obtained through bioconversion by *Lactobacillus arizonensis* of *Simmondsia chinensis.*

4. The cosmetic active substance according to one of claims 1 to 3, **characterized in that** it comprises sugars.

5. The cosmetic active substance according to the preceding claim, **characterized in that** the carbohydrate fraction of the active substance comprises polysaccharides and oligosaccharides of glucose, galactose and fructose.

6. The active substance according to one of the preceding claims, **characterized in that** it comprises at least one cyclic polyol.

7. The cosmetic active substance according to one of the preceding claims, **characterized in that** it comprises at least Pinitol.

8. The cosmetic active substance according to one of the preceding claims, **characterized in that** it comprises proteins and/or minerals and/or lactic acid.

9. The active substance according to one of the preceding claims, **characterized in that** it is in powder form.

10. A cosmetic composition comprising at least 0.1% by weight of an active substance according to one of claims 1 to 8.

11. The cosmetic composition according to the preceding claim, **characterized in that** it is in the form of a gel, emulsion or cream.

12. A method for obtaining an active substance according to one of claims 1 to 8, **characterized in that** it comprises the following steps:
- Culturing of *Lactobacillus arizonensis* in a culture medium supplemented with *Simmondsia chinensis*
- Stopping cultivation through thermal inactivation
- Separating the biomass from the supernatant
- Filtering the supernatant.

13. The method according to the preceding claim, **characterized in that** it also comprises a purification step and/or a decoloration step and/or a deodorization step and/or an atomization step.

14. Cosmetic use of a cosmetic active substance according to one of claims 1 to 8 on healthy skin to improve the beauty of the skin.

15. The cosmetic use of a cosmetic composition according to one of claims 10 or 11, on healthy skin, in order to improve the beauty of the skin.

16. The non-therapeutic cosmetic use of a cosmetic composition according to one of claims 10 or 11, for improving the glow of the skin and/or enhancing the radiance of the skin and/or increasing the hydration of the skin.

17. The cosmetic composition according to one of claims 10 or 11 for its use to regulate the balance of the skin flora and/or to reinforce the integrity of the skin barrier and/or to activate epidermal renewals and/or to energize the innate immune defenses.

18. A method for the cosmetic treatment of healthy skin in order to improve the beauty of the skin, **characterized in that** it consists in applying a composition according to one of claims 10 or 11 to the skin at least once a day.
